Europäisches Patentamt

European Patent Office

Office européen des brevets

(11) Publication number: **0 323 892 B1**

# EUROPEAN PATENT SPECIFICATION

(45) Date of publication of patent specification: **25.05.94**   (51) Int. Cl.⁵: **C01B 33/34**, B01J 29/28, C07C 5/41, C07C 5/22

(21) Application number: **89300036.4**

(22) Date of filing: **04.01.89**

(54) **Zeolite L.**

(30) Priority: **04.01.88 GB 8800046**

(43) Date of publication of application:
**12.07.89 Bulletin 89/28**

(45) Publication of the grant of the patent:
**25.05.94 Bulletin 94/21**

(84) Designated Contracting States:
**AT BE CH DE ES FR GB GR IT LI LU NL SE**

(56) References cited:
EP-A- 0 096 479
EP-A- 0 142 347
EP-A- 0 142 353
EP-A- 0 142 355
DE-A- 2 524 484

(73) Proprietor: **EXXON CHEMICAL PATENTS INC.**
**1900 East Linden Avenue**
**Linden, New Jersey 07036-0710(US)**

(72) Inventor: **Verduijn, Johannes Petrus**
**Westersingel 34**
**3202 XL Spijkenisse(NL)**

(74) Representative: **Bawden, Peter Charles**
**EXXON CHEMICAL LIMITED**
**EXXON CHEMICAL TECHNOLOGY CENTRE**
**PO Box 1**
**Abingdon Oxfordshire OX13 6BB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

This invention relates to the preparation of zeolite L and its use in catalysis, particularly for aromatization.

Zeolite L has been known for some time as an adsorbent, and in US-A-3 216 789 is described as an aluminosilicate of the formula:

$$0.9 - 1.3\ M_{2/n}O: Al_2O_3: 5.2 - 6.9\ SiO_2: yH_2O$$

(where M is an exchangeable cation of valency n and y is from 0 to 9) having a characteristic X-ray diffraction pattern. The preparation of zeolite L is described in US-A-3 216 789, EP-A-167755, EP-A-142355, EP-A-142347, EP-A-142349, EP-A-109199, PL-A-72149, US-A-3867512, and SU-548567.

EP-A-142355 describes a zeolite L prepared in a process employing a mother liquor recycle by adjusting the alkalinity of the crystallisation gel containing the mother liquor to suppress zeolite W formation and optionally heat treating the mother liquor prior to recycle to remove zeolite W nuclei and/or crystallites. The technique may be used to prepare cylindrical crystals of zeolite L of formula:

$$(0.9 - 1.3)M_{2/n}O: Al_2O_3: x\ SiO_2$$

where M is a cation of valence n, and x is from 5 to 7.5, preferably 5.7 to about 7.4.

EP-A-96479 describes and claims zeolite L having a characteristic morphology and size, which is particularly valuable for use as a catalyst base in hydrocarbon conversions such as aromatization, and comprising crystallites in the form of cylinders with a mean diameter of at least 0.1 micron, preferably at least 0.5 micron.

EP 96479 describes a synthesis of zeolite L which is conducted so that the amount of the contaminant zeolite W, which is know to grow in such a system as a competitive phase, is minimised. A preferred synthesis gel described in EP 96479 has the following mole ratios:

$$2.62K_2O:Al_2O_3:10SiO_2:16OH_2O$$

and it is discussed how this gel may be varied by changing the molar amount of one component within the following ranges:

$$
\begin{aligned}
K_2O: &\quad 2.4 - 3.0\ \text{moles} \\
Al_2O_3: &\quad 0.6 - 1.3\ \text{moles} \\
SiO_2: &\quad 8 - 12\ \text{moles} \\
H_2O: &\quad 120 - 240\ \text{moles}
\end{aligned}
$$

EP-A-142353, EP-A-142354 and EP-A-185519 describe developments of this process for forming cylindrical zeolite L.

Zeolite L may be used as a catalyst base in aromatisation reactions. US 4 104 320 discloses dehydrocyclisation of aliphatic compounds in the presence of hydrogen using a catalyst comprising zeolite L and a group VIII metal. The particular zeolite disclosed in EP 96479 is remarkably effective in such aromatisation reactions being capable of forming catalysts which have extended lifetime. Such dehydrocyclisation and/or aromatisation reactions and catalysts for use in such reactions are also described in EP-A-107389, EP-A-184451, EP-A-142351, EP-A-145289, EP-A-184450, US-A-4614834, GB-A-2116450, GB-A-2114150, US-A-4458025, US-A-4456527, GB-A-2142648, GB-A-2106483, US-A-4443326, GB-A-2121427, GB-A-2153840, GB-A-2153384, US-A-4517306, US-A-4539304, US-A-4539305, US-A-4547472, GB-A-2166972, US-A-4579831, US-A-4608356 and EP-A-201856.

The product recovered from the usual methods used to prepare zeolite L is a fine sized crystalline material. Several of the uses as catalysts or molecular sieves require a product in a size range substantially larger than the size of the product recovered from the preparation processes of the prior art. To meet this demand, various binders are used in forming steps to prepare aggregates containing zeolite L as the principal ingredient such as pellets, extrudates, or tablets. These aggregates lose some of their activity per unit weight since the binder has a different and low activity and acts as a diluent of the molecular sieve

2

EP 0 323 892 B1

activity and the conventionally-bound aggregates frequently do not have sufficient crushing strength, particularly when they contain the cylindrical zeolite L crystallites as described in EP-A-96479. In addition particles made using alumina as binder are susceptible to blocking of the zeolite pores, as a result of alumina migration. It is therefore highly desirable to develop a method of preparing binderless aggregates having a particle size suitable for catalyst or sieve systems and possessing good attrition resistance.

The prior art has developed processes of producing binderless sieve aggregates from silica and alumina starting materials such as silica-alumina catalysts and clay. Unfortunately the products produced by these processes, especially where clay is used as a starting material, generally have very poor attrition resistance and thus rapidly break during use into unsuitable powders which must be replaced.

US-A-3 650 687 describes processes for the preparation of binderless zeolite particles including zeolite L, in which an alumina silicate clay is slurried with an alkali silicate, spray dried to form particles of the desired finished size and then treated with an alkali and aged to convert the particles to zeolite. In an alternative, a hydrated clay is slurried and spray dried to form particles, then calcined and reacted with the other components necessary to form a zeolite. Thus zeolite is only formed after the final particles have been formed. Predictable formation of zeolite having optimum catalytic properties may be difficult under such circumstances.

Also spray drying can be used only to give small particles, typically of 100 to 400 microns, which are only suitable for fluidized beds whereas reactors usually need particle sizes of at least 0.8 mm, preferably at least 1.5 mm and typically 3 mm.

GB 1316311 describes binderless zeolite particles which may be of zeolite L, and which are formed by pelleting, crush and repelleting repeatedly to give products of the desired strength. This is a time-consuming procedure which is costly and can damage the zeolite crystals.

GB 2109359 describes preparation of zeolite 3A and 4A binderless particles in various processes in which kaolin clay and sodium hydroxide (in some cases with zeolite) are formed into beads and then reacted with further sodium hydroxide to form zeolite 4A (sodium form) which is exchanged to form zeolite 3A (potassium form). It is clearly stated that direct formation of a potassium zeolite is not possible in this process.

GB 2160517 describes the formation of so called preformed zeolite particles, which may be zeolite L particles prepared from a starting material, which may be a synthetic zeolite but must have a silica/alumina ratio lower than the product. The starting material is reacted with a silica material and an alkali to form the product. To form zeolite L either zeolite 3A, kaolin or a silica-alumina starting material is used. The products are necessarily more silica rich than the starting zeolite. This process has practical handling problems in treating particles with a silica containing material.

The prior art systems are either unsuitable for providing binderless particles of zeolite L having high catalytic or absorbent activity, or result in particles of inadequate strength to be suitable for handling in practical applications or are impractical for large scale operation.

It has been found that the potassium form of zeolite L hereinafter identified as zeolite KL shows enhanced properties as an aromatisation catalyst if it also contains some caesium. However attempts to replace some of the potassium ions by caesium ions in the preparation of zeolite KL have not been very successful in the past as the pressure of caesium ions favours the formation of pollucite rather than zeolite L. We have now found a method of incorporating caesium in binder-free particles of the zeolite KL without any substantial formation of pollucite.

According to this invention binder-free shaped particles of caesium containing zeolite KL are prepared by a process comprising forming a mixture of shaped particles of silica-bound zeolite KL with an aqueous solution containing sources of caesium, potassium and aluminium and obtaining the binder-free caesium-containing shaped particles of zeolite KL by crystallisation of the mixture. In a modification of this process one can use the shaped particles of zeolite KL after they have been ground to finer particles of silica-bound zeolite KL. One then obtains a binder-free caesium-containing product which is also in ground form.

The binder-free particles obtained by the process of this invention have excellent physical strength without undesirable loss of catalytic performance and/or capacity.

When used herein in relation to the invention the terms "binderless" or "binder-free" refer to a plurality of individual zeolite L containing particles held together without the use of a significant amount of non-zeolitic binder. Preferably the particles contain less than 10 wt % (based on the weight of the total particles) of non-zeolitic binder. More preferably, the particles contain less than 5 wt % of non-zeolitic binders, and it is most preferable for the particles to be substantially free of non-zeolitic binder.

The shaped particles of zeolite KL may take various forms and are usually formed by extrusion to produce for example tablets or pellets.

3

The zeolite KL starting material is silica bound and this means that the binder is silica. The silica bound zeolite KL is usually derived from zeolite KL crystallites.

The zeolite L crystallites used as starting material may be prepared by any of the known procedures, such as those described in the patents identified hereinbefore as relating to zeolite L preparation. However, it is highly preferred that the zeolite L should be in a form having beneficial effects on the catalytic performance of the zeolite. The starting material may be a zeolite comprising crystallites in the form of cylinders with a mean diameter of at least 0.05 micron, preferably at least 0.1 micron, typically at least 0.5 micron, such as described in EP-A-96479).

The zeolite KL is preferably an aluminosilicate and will be described hereinafter in terms of being an aluminosilicate, though other elemental substitutions are possible, for example aluminium may be substituted by gallium, boron, iron and similar trivalent elements, and silicon may be substituted by elements such as germanium or phosphorus. The aluminosilicates preferably have a composition (expressed in terms of molar ratios of the constituent oxides in anhydrous form) of:

$$(0.9 - 1.3) \ K_2O: Al_2O_3: xSiO_2 \qquad (I)$$

wherein x is from 4 to 7.5. The zeolite L preferably has high crystallinity as shown by a well defined X-ray diffraction pattern with sharp peaks.

The zeolite KL may be hydrated, typically with from 0 to 9 moles of water per mole of $Al_2O_3$.

To make the silica bound zeolite KL, the zeolite KL crystals are thoroughly mixed with silica gel and preferably at a later stage in the mixing, methocel (hydroxypropyl methyl cellulose extrusion aid) is added and a very thick and smooth paste is obtained. The resulting paste usually can be collected and later extruded or otherwise shaped. The shaped particles eg extrudates, can then be dried to remove residual water and calcined in air.

The shaped particles of silica bound zeolite KL are mixed with an aqueous solution containing sources of caesium, potassium and aluminium, preferably a caesium-containing potassium aluminate solution. This solution should preferably have a mole ratio (expressed in terms of the oxides) of $K_2O/(K_2O + Cs_2O)$ of at least 0.8.

The overall synthesis mixture expressed as moles of oxide except for caesium which is expressed as moles of CsX where X is halogen should preferably be:

2.50 to 3.20 $K_2O$/0.5 to 1.00 $CsX$/1.30 to 2.50 $Al_2O_3$/10 $SiO_2$/110 to 170 $H_2O$

However when $CsOH.H_2O$ is used the overall synthesis mixture is preferably:

2.25 to 2.70 $K_2O$/0.025 to 0.50 $Cs_2O$/1.30 to 2.50 $Al_2O_3$/10 $SiO_2$/110 to 170 $H_2O$

The crystallisation is generally carried out in a sealed autoclave and thus at autogenous pressure. It is generally inconvenient, although possible to employ higher pressures. Lower pressure (and lower temperature) will require longer crystallisation times.

Crystallisation time is related to the crystallisation temperature. The crystallisation is preferably carried out at from 100°C to 200°C and at this temperature the crystallisation time may be from 15 to 96 hours, typically from 48 to 72 hours, with shorter times being possible with higher temperatures and/or higher alkalinity ($K_2O/SiO_2$). Lower temperatures may require much longer times and may also require adjustment of alkalinity to achieve good yield of the desired product, whereas times of less than 24 hours are possible when higher temperatures are used.

After crystallisation the shaped particles should be washed with successive portions of water until the pH of the last wash water is between 9 and 10, e.g. about 9.5. The shaped particles should then be dried at a temperature of 130°C to 170°C, e.g. about 15 hours at 150°C.

X-ray diffraction (XRD) shows that the product of this invention consists of pure zeolite L and that the crystallinity increase versus the silica bound shaped particles of zeolite KL (the starting material) is up to 30%. This XRD-crystallinity increase is somewhat lower than in a similar synthesis wherein no caesium is used and this is an indication that the binder-free product contains caesium.

If the starting material is in the form of crushed particles the final product is also particulate. However this particulate product can be turned into shaped particles by use of conventional binder techniques, by the binderless process disclosed in the specification of our patent application GB 8704365 or by the present process.

It has been found that the binderless zeolite L particles prepared by the process of the invention are excellent catalyst bases and may be used in conjunction with one or more catalytically-active metals in a wide variety of catalytic reactions. The particular morphology of the crystals appears to result in a particular stable base for catalytically active metals with a surprising resistance to metal catalyst deactivation. Also the particles have increased particle strength. In addition, they have displayed low acidity which makes them especially suited to catalytic applications where a low acid site strength is advantageous such as aromatisation.

The catalytically-active metal(s) may be, for example, a Group VIII metal such as platinum, or tin or germanium as described in US-A-4104320, or a combination of platinum and rhenium as described in GB-A-2004764 or BE-A-888365. In the latter case, the catalyst may for appropriate circumstances also incorporate halogen as described in US-A-4165276, silver as described in US-A-4295959 and US-A-4206040, cadmium as described in US-A-4295960 and US-A-4231897 or sulphur as described in GB-A-1600927.

A particularly advantageous catalyst composition incorporates from 0.1 to 6.0 wt.%, (based on the total weight of the composition), preferably from 0.1 to 1.5 wt.% platinum or palladium, since this gives excellent results in aromatisation. From 0.4 to 1.2 wt.% platinum is particularly preferred. Accordingly the invention provides a catalyst comprising the binder-free zeolite obtained by the process of this invention and a catalytically-active metal.

The zeolite L product of the process of this invention may be used in a process for the conversion of a hydrocarbon feed in which the feed is contacted with a catalyst as described above under appropriate conditions to bring about the desired conversion. They may, for example, be useful in reactions involving aromatisation and/or dehydrocyclisation and/or isomerisation and/or dehydrogenation reaction. They are particularly useful in a process for the dehydrocyclisation and/or isomerisation of aliphatic hydrocarbons in which the hydrocarbons are contacted at a temperature of from 370 to 600°C, preferably 430 to 550°C, with a catalyst comprising zeolite L of the invention, and preferably incorporating at least one Group VIII metal having dehydrogenating activity, so as to convert at least part of the aliphatic hydrocarbons into aromatic hydrocarbons.

The aliphatic hydrocarbons may be straight or branched chain acyclic hydrocarbons, and particularly paraffins such as hexane, although mixtures of hydrocarbons may also be used such as paraffin fractions containing a range of alkanes possibly with minor amounts of other hydrocarbons. Cycloaliphatic hydrocarbon such as methylcyclopentane may also be used. In a preferred embodiment the feed to a process for preparing aromatic hydrocarbons and particularly benzene comprises hexanes. The temperature of the catalytic reaction may be from 370 to 600°C, preferably 430 to 550°C and preferably pressures in excess of atmospheric are used, for example up to 2000 KPa, more preferably 500 to 1000 KPa. Hydrogen is employed in the formation of aromatic hydrocarbons preferably with a hydrogen to feed ratio of less than 10.

The process is preferably otherwise carried out in the manner described in US 4104320, BE 888365, EP 0040119, EP 0142351, EP 0145289 or EP 0142352.

The invention is now described by the the following Examples.

Example 1

A synthesis mixture was obtained by the mixing of two solutions - Solution A and Solution B - and the addition of extrudates of silica-bound zeolite KL. The weight of the reactant given in grams were as follows:

Solution A: Potassium aluminate solution:

$$\text{KOH pellets (87.3\% purity)} \qquad : \quad 7.376$$
$$\text{Al(OH)}_3 \text{ powder (purity 98.6\%)} \qquad : \quad 5.116$$
$$\text{H}_2\text{O} \qquad : \quad 16.46$$
$$\text{Additional H}_2\text{O} \qquad : \quad 5.89$$

The aluminium trioxide was dissolved by boiling and after cooling to ambient temperature the weight loss due to evaporation of water was corrected.

Solution B: CsCl

```
CsCl (≥99% purity)                    :  1.679
H₂O                                   : 15.00
Additional H₂O                        : 10.00
```

The extrudates were prepared by the process according to Example 1b of our patent application GB 8704365.

Solution B was added to Solution A, the additional water being used to rinse the beaker containing Solution B. The combined Solution A/B was poured into a 300 ml stainless steel autoclave together with the 5.89 g additional water which was used to rinse the beaker containing Solution A/B. Next the extrudates of silica-bound zeolite KL were added to the contents of the autoclave.

The overall composition of the synthesis mixture (expressed as oxides) was:

2.89 $K_2O$/0.50 CsCl/1.63 $Al_2O_3$/10 $SiO_2$*/141 $H_2O$

Crystallisation:

The autoclave was heated up over 6 hrs to 175°C and kept at this temperature for 65 hrs.

Washing and drying:

The extrudates were washed with 8 portions of water until the pH of the last washwater was 9.3. The total amount of water used was 3200 ml, the total washing time was 38 hrs. The extrudates were dried for 16 hrs at 150°C. The amount of extrudates recovered was 44.6 grams. The extrudates had an excellent physical strength having a crush strength of 3.12 lb/mm (1.42 kg/mm). This compares with crush strengths of conventional extrudates of about 1.07 lb/mm (0.49 kg/mm).

Properties of Extrudates:

Toluene adsorption capacity at 30°C and at $p/p_o$ = 0.25.

| wt% toluene | | |
|---|---|---|
| adsorbed | desorbed | micropore capacity |
| 9.06 | 0.05 | 9.01 |

Chemical Analysis:   Al 10.9 wt%, K 13.4 wt%
Cs 2.4 wt % and Si 26.7 wt%

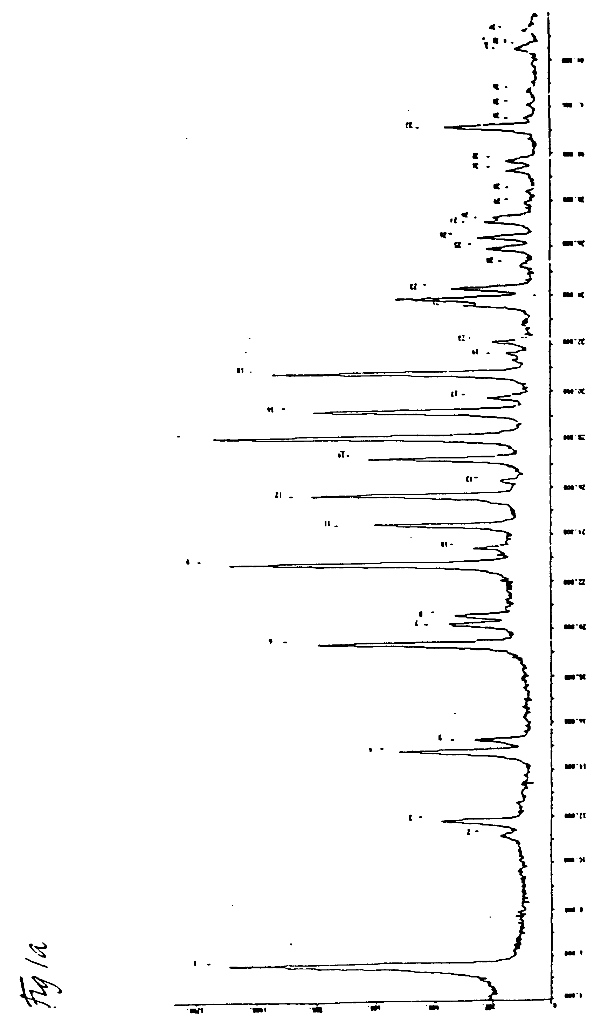

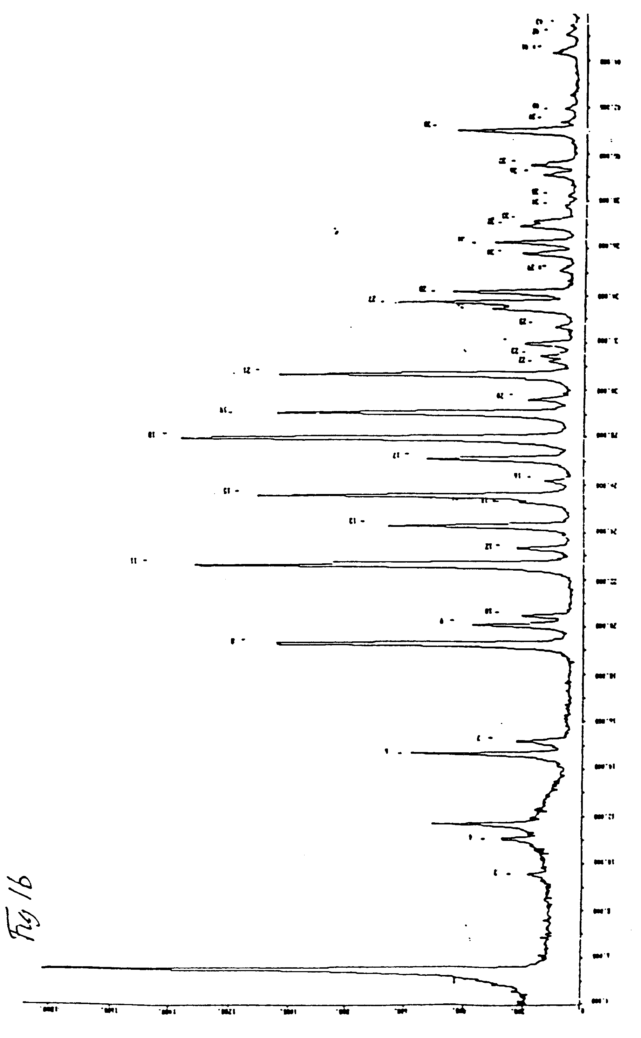

X-ray diffraction shoved that the product consisted of pure zeolite L, the crystallinity increase versus the silica-bound KL extrudates was 20%. The apparent XRD-crystallinity increase was somewhat lower than in similar synthesis in which no caesium is used (30% increase). This is an indication that the binder-free product contains caesium. Comparative diffractrograms of the starting silica-bound extrudates and of the binder-free product are shown in Fig. 1a and 1b respectively.

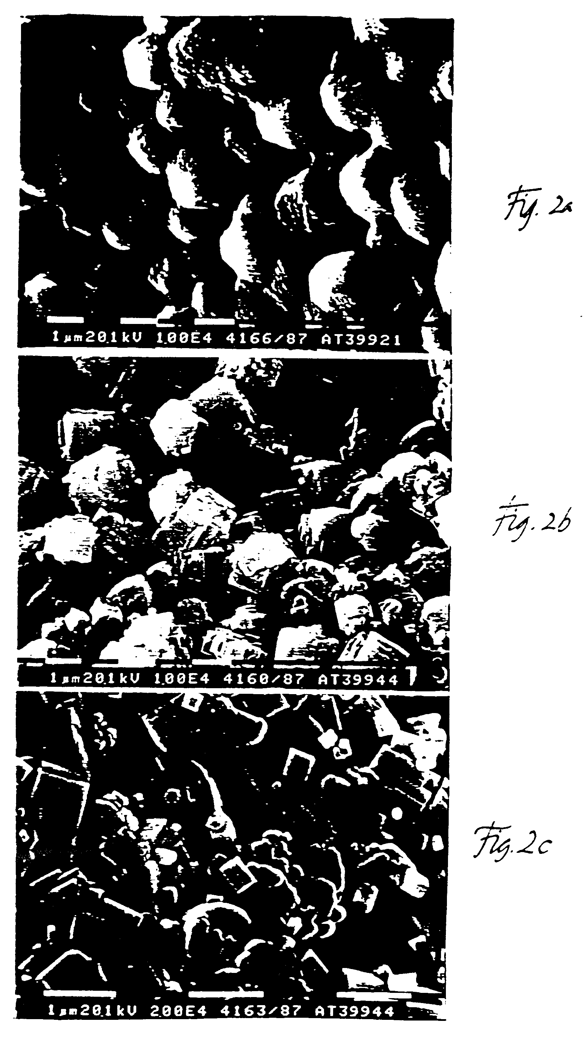

SEM (scanning electron micrographs) showed that the amorphous silica binder was completely converted into zeolite L. Comparative 10000 * SEM micrographs of the starting silica-bound extrudate and the binder-free material are given in Figs. 2a, b and c in which Fig. 2a shows the starting silica-bound zeolite KL extrudate with an enlargement of 10000, Fig. 2b shows the binder-free zeolite KL product extrudate with an enlargement of 10000 and Fig. 2c is the same as Fig. 2b but with an enlargement of

* Silica present as binder in the extrudate.

20,000.

Example 2

In this Example binder free KL extrudates in the presence of caesium were prepared. In the two syntheses the Cs concentration was increased from 0.50 $Cs^+/10$ $SiO_2$ to 1.00 $Cs^+/10$ $SiO_2$ and the Cs-source was CsCl in one case and CsOH in the other case.

For the preparation of the first binderless KL extrudate the synthesis mixtures (weight reactants in grams) were:

| | | | |
|---|---|---|---|
| Solution A | KOH pellets (86.8%) | 13.903 | aluminate |
| | $Al(OH)_3$ (98.6%) | 9.592 | solution |
| | $H_2O$ | 30.87 | |
| | Additional water | 12.28 | |

| | | | |
|---|---|---|---|
| Solution B | CsCl powder (≧99%) | 6.304 | CsCl |
| | $H_2O$ | 27.98 | solution |
| | Additional (rinsing) water | 18.47 | |
| | Silica bound extrudates | 75.00 | |

After cooling to ambient temperature Solution A was poured into a 300 ml stainless steel autoclave. The beaker which contained the aluminate solution was rinsed with the additional water; this water was poured into the autoclave. The CsCl solution was poured into the autoclave together with the rinsing water. The contents of the autoclave were mixed. Next the silica bound KL extrudates were added to the contents of the autoclave. The composition (moles) of the synthesis mixture was:

2.87 $K_2O$/1.00 CsCl/1.62 $Al_2O_3$/10 $SiO_2$*/141 $H_2O$

The mixture was then crystallised with a heat up rate of approx. 5 hours to 175°C and the mixture was held at 175°C for 65 hours.

Thereafter the extrudates were prewashed with 500ml water, followed by 9 further washings. The total amount of water was 3600 ml and total washing time 25 hours. The pH of the last wash water was 9.2. The extrudates were dried overnight at 150°C, and the weight of dry extrudates recovered was 87.1 grams. The weight increase of starting Si-bound extrudates was 16.1%.

XRD showed that the product was substantially pure zeolite L.

For the preparation of the second binderless KL extrudate the Cs-source was $CsOH.H_2O$. In this synthesis the Cs-source also provides part of the alkalinity; therefore the potassium content was adjusted to give an alkalinity $(K_2O + Cs_2O)/10$ $SiO_2$ of 2.86.

* amorphous binder

7

The preparation synthesis mixture (weight reactants in grams):

$$A \begin{cases} \text{KOH pellets (86.8\%)} & 11.419 \\ \text{CsOH.H}_2\text{O} \; (\geq 99\%) & 6.286 \\ \text{Al(OH)}_3 \; (98.6\%) & 9.596 \\ \text{H}_2\text{O} & 30.88 \end{cases} \quad \text{potassium-caesium aluminate solution}$$

| | |
|---|---|
| Additional (rinsing) water | 59.94 |
| Silica bound extrudates | 75.02 |

After cooling solution A was poured into a 300 ml stainless steel autoclave together with the rinsing water. After mixing the contents of the autoclave the Si-bound extrudates were added.

The composition (moles) of the synthesis mixture was:

2.36 $K_2O$/0.50 $Cs_2O$/1.62 $Al_2O_3$/10 $SiO_2$/142 $H_2O$ ($K_2O + Cs_2O$)/10 $SiO_2$ = 2.86

The mixture was then crystallised with a heat up rate of approx. 4 hours to 175°C and the mixture was held at 175°C for 65 hours. Thereafter the extrudates were prewashed with 500ml of water, followed by further washings, the total amount of washwater was 3200 ml. The pH of the last wash water was 9.3. The extrudates were dried overnight at 150°C and the weight of the extrudates recovered was 87.2 grams. The weight increase of the starting Si-bound extrudates was 16.2%. XRD showed that the product was substantially pure zeolite L.

**Claims**

1. A process for preparing binder-free shaped particles of caesium-containing zeolite KL which comprises forming a mixture of shaped particles of silica bound zeolite KL with an aqueous solution containing sources of caesium, potassium and aluminium and obtaining the binder-free caesium-containing shaped particles of zeolite KL by crystallisation of the mixture.

2. A process according to claim 1 in which the shaped particles of silica-bound zeolite KL are derived from zeolite KL crystallites.

3. A process according to either of claims 1 and 2 wherein the aqueous solution containing sources of caesium, potassium and aluminium is a caesium-containing potassium aluminate solution.

4. A process according to any one of the preceding claims wherein the overall synthesis mixture expressed as moles of oxide except for caesium which is expressed as moles of CsX where X is halogen is:

   2.50 to 3.20 $K_2O$/0.05 to 1.00 CsX/1.30 to 2.50 $Al_2O_3$/10 $SiO_2$/110 to 170 $H_2O$, or where $CsOH.H_2O$ is used 2.25 - 2.70$K_2O$/0.025 - 0.50$Cs_2O$/1.30 - 2.50 $Al_2O_3$/10$SiO_2$/110 - 170$H_2O$

5. A process according to any one of the preceeding claims wherein the mole ratio (expressed in terms of the oxide) of $K_2O/K_2O + Cs_2O$ is at least 0.8.

6. A modification of the process according to any one of the preceding claims wherein the shaped particles of silica-bound zeolite KL are ground into finer particles of silica bound zeolite KL before being mixed with eh aqueous solution containing sources of caesium, potassium and aluminium.

7. A process for preparing a catalyst comprising combining a catalytically active metal and the binder-free particles of caesium-containing zeolite KL prepared by a process according to any one of the preceding claims.

8. A process according to claim 7 wherein the metal is platinum or palladium in an amount of 0.1 to 6.0 weight % based on the total weight of the catalyst.

9. A process for the dehydrocyclization and/or isomerisation of an aliphatic hydrocarbon in which the aliphatic hydrocarbon is contacted at a temperature of from 370°C to 600°C with the catalyst prepared according to either of claims 7 or 8 so as to convert at least part of the aliphatic hydrocarbon into an aromatic hydrocarbon.

**Patentansprüche**

1. Verfahren zur Herstellung von bindemittelfreien geformten Teilchen aus cäsiumhaltigem Zeolith KL, bei dem eine Mischung aus geformten Teilchen aus mit Siliciumdioxid gebundenem Zeolith KL und einer wäßrigen Lösung gebildet wird, die Cäsium-, Kalium- und Aluminiumquellen enthält, und die bindemittelfreien cäsiumhaltigen geformten Teilchen aus Zeolith KL durch Kristallisation der Mischung erhalten werden.

2. Verfahren nach Anspruch 1, bei dem die geformten Teilchen aus mit Siliciumdioxid gebundenem Zeolith KL von Zeolith-KL-Kristalliten abgeleitet sind.

3. Verfahren nach einem der Ansprüche 1 oder 2, bei dem die wäßrige Lösung, die Cäsium-, Kalium- und Aluminiumquellen enthält, eine cäsiumhaltige Kaliumaluminatlösung ist.

4. Verfahren nach einem der vorhergehenden Ansprüche, bei dem die gesamte Synthesemischung ausgedrückt als Mole an Oxid, mit Ausnahme von Cäsium, das als Mol von CsX, wobei X Halogen ist, ausgedrückt wird,
2,50 bis 3,20 $K_2O$/0,05 bis 1,00 CsX/1,30 bis 2,50 $Al_2O_3$/10 $SiO_2$/110 bis 170 $H_2O$ oder wenn $CsOH \cdot H_2O$ verwendet wird,
2,25 bis 2,70 $K_2O$/0,025 bis 0,50 $Cs_2O$/1,30 bis 2,50 $Al_2O_3$/10 $SiO_2$/110 bis 170 $H_2O$
ist.

5. Verfahren nach einem der vorhergehenden Ansprüche, bei dem das Molverhältnis (ausgedrückt als Oxide) von $K_2O$/($K_2O$ + $Cs_2O$) mindestens 0,8 beträgt.

6. Veränderung des Verfahrens gemäß einem der vorhergehenden Ansprüche, bei dem die geformten Teilchen aus mit Siliciumdioxid gebundenem Zeolith KL zu feineren Teilchen aus mit Siliciumdioxid gebundenem Zeolith KL gemahlen werden, bevor sie mit der wäßrigen Lösung gemischt werden, die Cäsium-, Kalium- und Aluminiumquellen enthält.

7. Verfahren zur Herstellung eines Katalysators, bei dem ein katalytisch aktives Metall und die nach einem Verfahren gemäß einem der vorhergehenden Ansprüche hergestellten bindemittelfreien Teilchen aus cäsiumhaltigem Zeolith KL kombiniert werden.

8. Verfahren nach Anspruch 7, bei dem das Metall Platin oder Palladium in einer Menge von 0,1 bis 6,0 Gew.% ist, bezogen auf das Gesamtgewicht des Katalysators.

9. Verfahren zur Dehydrocyclisierung und/oder Isomerisierung eines aliphatischen Kohlenwasserstoffs, bei dem der aliphatische Kohlenwasserstoff bei einer Temperatur von 370°C bis 600°C mit dem gemäß Anspruch 7 oder 8 hergestellten Katalysator kontaktiert wird, so daß mindestens ein Teil des aliphatischen Kohlenwasserstoffs in einen aromatischen Kohlenwasserstoff umgewandelt wird.

**Revendications**

1. Procédé de préparation de particules façonnées, dépourvues de liant, de zéolite KL contenant du césium, qui comprend la formation d'un mélange de particules façonnées de zéolite KL, liée avec de la silice, avec une solution aqueuse contenant des sources de césium, de potassium et d'aluminium, et l'obtention des particules façonnées de zéolite KL contenant du césium, dépourvues de liant, par cristallisation du mélange.

**2.** Procédé suivant la revendication 1, dans lequel les particules façonnées de zéolite KL liée avec de la silice sont obtenues à partir de cristallites de zéolite KL.

**3.** Procédé suivant chacune des revendications 1 et 2, dans lequel la solution aqueuse contenant des sources de césium, de potassium et d'aluminium est une solution d'aluminate de potassium contenant du césium.

**4.** Procédé suivant l'une quelconque des revendications précédentes, dans lequel le mélange total de synthèse, exprimé en moles d'oxyde à l'exception du césium qui est exprimé en moles de CsX dans lequel X représente un halogène, est :
2,50 à 3,20 K$_2$/0,05 à 1,00 CsX/1,30 à 2,50 Al$_2$O$_3$/10 SiO$_2$/110 à 170 H$_2$O, ou, lorsque du CsOH.H$_2$O est utilisé, 2,25 à 2,70 K$_2$O/0,025 à 0,50 Cs$_2$O/1,30 à 2,50 Al$_2$O$_3$/10 SiO$_2$/110 à 170 H$_2$O.

**5.** Procédé suivant l'une quelconque des revendications précédentes, dans lequel le rapport molaire (exprimé en termes de l'oxyde) K$_2$O/K$_2$O + Cs$_2$O est au moins égal à 0,8.

**6.** Modification du procédé suivant l'une quelconque des revendications précédentes, dans lequel les particules façonnées de zéolite KL liée avec de la silice sont broyées en particules plus fines de zéolite KL liée avec de la silice avant leur mélange à la solution aqueuse contenant des sources de césium, de potassium et d'aluminium.

**7.** Procédé de préparation d'un catalyseur, comprenant le mélange d'un métal catalytiquement actif et des particules dépourvues de liant de zéolite KL contenant du césium préparées par un procédé suivant l'une quelconque des revendications précédentes.

**8.** Procédé suivant la revendication 7, dans lequel le métal et le platine ou le palladium en une quantité de 0,1 à 6,0 % en poids sur la base du poids total du catalyseur.

**9.** Procédé de déshydrocyclisation et/ou d'isomérisation d'un hydrocarbure aliphatique, dans lequel l'hydrocarbure aliphatique est mis en contact à une température de 370°C à 600°C avec le catalyseur préparé suivant chacune des revendications 7 et 8 de manière à transformer au moins une partie de l'hydrocarbure aliphatique en un hydrocarbure aromatique.

Fig. 1a

Fig 1b

Fig. 2a

1 µm 20.1kV 1.00E4 4166/87 AT39921

Fig. 2b

1 µm 20.1kV 1.00E4 4160/87 AT39944

Fig. 2c

1 µm 20.1kV 2.00E4 4163/87 AT39944